# EUROPEAN PATENT APPLICATION

(11) **EP 4 625 422 A1**
(43) Date of publication of application: **01.10.2025**
(21) Application number: 23898393.6
(22) Date of filing: 01.12.2023
(51) Int. Cl.: G16C 20/30, G16C 20/80, G16C 20/70, G16C 20/20, G06N 3/02

(54) **LINE CONNECTION-TYPE OBJECT PREDICTION DEVICE AND METHOD USING ARTIFICIAL INTELLIGENCE**

(30) Priority: 01.12.2022 KR 20220166081
(71) Applicant: LG Management Development Institute Co., Ltd., Seoul 07336 (KR)
(72) Inventor: JO, Ah Ra, Gimpo-si, Gyeonggi-do 10113 (KR); JO, Yeon Sik, Seoul 07791 (KR); LEE, Seung Jun, Seoul 04182 (KR); LEE, Soon Young, Seoul 06299 (KR)
(74) Representative: BCKIP Part mbB
(86) International application number: PCT/KR2023/019707
(87) International publication number: WO 2024/117870

(57) **Abstract**

The present disclosure is characterized in that objects are detected using atoms and bonds, which make up a molecular structural formula, as nodes and edges, respectively, when recognizing a molecular structural formula image representing the molecular structure of a compound, and the detection information about nodes is used when detecting edges for bonds.

## Description

### [TECHNICAL FIELD]

The present disclosure relates to a line connection-type object prediction device. More specifically, the present disclosure relates to a line connection-type object prediction device and method using artificial intelligence.

### [BACKGROUND ART]

In general, conventional line connection-type object prediction devices predicted result information on the state of connecting objects with lines in an image.

This line connection-type object prediction device predicted result information on the state of connecting objects in a map with lines, result information on the state of connecting objects in a drawing with lines, and result information on the state of connecting objects in a structural formula with lines.

For example, the structural formula of a compound is a pictorial representation of its molecular structure, showing how the atoms are arranged. The structural formula of a compound may either explicitly or implicitly represent the chemical bonds within the molecule.

Unlike chemical formulas or chemical names, structural formulas may represent molecular structures. Accordingly, chemical reactions or syntheses are explained using structural formulas rather than chemical names, because the structural formulas may visualize the changes that occur in molecules.

Many compounds have different structures, but exist as isomers with the same overall chemical formula. Accordingly, a structural formula represents an arrangement of atoms that may not be represented by a chemical formula.

However, conventional line connection-type object prediction devices had limitations in reducing errors in predicting bonds as atoms when line connection-type objects are structural formulas, and have limitations in improving the analysis accuracy and computation speed of structural formulas.

Accordingly, recently, research has been continuously conducted on improved line connection-type object prediction devices that may reduce prediction errors and improve analysis accuracy and computation speed when the state of objects connected by lines is predicted.

(Related Art Document 1) Korean Patent Application Publication No. 10-2022-0112692 (published on August 11, 2022)

### [DETAILED DESCRIPTION OF INVENTION]

### [TECHNICAL PROBLEMS]

An aspect of the embodiment disclosed in the present disclosure is directed to improving analysis accuracy and computation speed while reducing prediction errors when the state of objects connected by lines is predicted.

The aspects of the present disclosure are not limited to those mentioned above, and other aspects not mentioned herein will be clearly understood by those skilled in the art from the following description.

### [TECHNICAL SOLUTION]

A line connection-type object prediction device using artificial intelligence according to an embodiment of the present disclosure includes: a memory; and a processor electrically connected to the memory and controlling an operation related to line connection-type object prediction,
wherein the processor is characterized by:
receiving an image including a molecular structural formula image;
detecting nodes corresponding to atoms of a molecular structural formula in the received image to generate node detection information; detecting edges corresponding to bonds of the molecular structural formula based on the node detection information and the received image to generate edge detection information; and outputting prediction result information of the molecular structural formula image including the node detection information and the edge detection information.

In this connection, the processor may output an entire feature map for the received image through a backbone network.

In addition, the processor may extract a plurality of candidate regions (region proposals) predicted as the nodes based on the output entire feature map using a region proposal network (RPN).

In addition, the processor may classify and detect the nodes based on the entire feature map and the extracted plurality of candidate regions using a region of interest (ROI) module and output the node detection information.

In addition, the ROI module may determine a positive sample for a node among the plurality of candidate regions through a proposal target layer.

In addition, the ROI module may perform RoI pooling based on the determined positive sample and the entire feature map to output a feature map of a fixed-size node for the positive sample.

In addition, the ROI module may input the feature map of the node into a fully connected layer to output a feature vector, classify a class for the output feature vector, and perform bounding box regressor to output the node detection information for the molecular structural formula image.

In addition, the processor may detect an edge based on the node detection information and the entire feature map using a line of interest (LOI) module, which is a CNN-based edge detector, and output the edge detection information.

The processor may:
datafy a graphical representation of the molecular structural formula based on object detection information including the node detection information and the edge detection information through a graphical reconstruction operator, and output the prediction result information of the molecular structural formula image.

In addition, the processor may perform an auxiliary task of feeding back the edge detection information output from the LOI module to the ROI module to perform node detection again.

In addition, the processor may train the LOI module, the ROI module, the region proposal network, and a backbone based on a training data set in which node labels and edge labels are set for training images including the molecular structural formula image.

In addition, the processor may train the LOI module, the ROI module, the region proposal network, and the backbone by simultaneously applying the node labels and the edge labels to the molecular structural formula image based on a unifying loss for the nodes and the edges.

In addition, the processor may further output edge classification information by classifying the edges by class of the edges through a segmentation module.

The edge classification information output above may be further included in the object detection information.

In addition, a computer-readable recording medium recording a computer program for executing a method for implementing the present disclosure may be further provided.

### [EFFECT OF INVENTION]

Analysis accuracy and computation speed can be improved while reducing prediction errors when the state of objects connected by lines is predicted.

The benefits of the present disclosure are not limited to those mentioned above, and other benefits not mentioned herein will be clearly understood by those skilled in the art from the following description.

### [BRIEF DESCRIPTION OF THE DRAWING]

FIG. 1 is a diagram illustrating a line connection-type object prediction system using artificial intelligence according to an embodiment of the present disclosure.
FIG. 2 illustrates the configuration of a line connection-type object prediction device of FIG. 1.
FIGS. 3 and 4A to 4C are diagrams illustrating an example of a process for outputting line connection-type object prediction result information through the processor of FIG. 2.
FIG. 5 is a flowchart illustrating a line connection-type object prediction method using artificial intelligence according to an embodiment of the present disclosure.
FIG. 6 is a diagram illustrating an example of a process for detecting atoms, bonds, and lines in the processor of FIG. 2.
FIGS. 7 to 13 are diagrams illustrating examples of a process for generating an artificial intelligence model based on linkage information between atoms, bonds, and lines in the processor of FIG. 2.

### [BEST MODE FOR CARRYING OUT THE INVENTION]

Like reference numerals refer to like elements throughout the specification of the present disclosure. This disclosure does not describe all elements of embodiments, and a general description in the technical field to which the present disclosure pertains or a repetitive description in the embodiments will be omitted. As used herein, the term "unit," "module," "member," or "block" may be implemented as software or hardware. Depending on embodiments, a plurality of "units," "modules," "members," or "blocks" may be implemented as one element, or one "unit," "module," "member," or "block" may include a plurality of elements.

Throughout the specification, when a part is referred to as being "connected" to another part, this includes not only a direct connection but also an indirect connection, wherein the indirect connection includes a connection via a wireless communication network.

Terms such as "first" and "second" may be used to distinguish one component from another, but the components are not restricted by the aforementioned terms.

Hereinafter, the operating principle and embodiments of the present disclosure are described with reference to the attached drawings.

In this specification, a line connection-type object prediction device using artificial intelligence according to an embodiment of the present disclosure includes various devices that may perform computation processing and provide results to a user. For example, the line connection-type object prediction device using the artificial intelligence according to an embodiment of the present disclosure may include all of a computer, a server device, and a portable terminal, or may be in the form of any one thereof.

Herein, the computer may include, for example, a notebook, desktop, laptop, tablet PC, slate PC, etc. equipped with a WEB Browser.

The server device is a server that processes information by communicating with an external device, and may include an application server, a computing server, a database server, a file server, a proxy server, and a web server.

The portable terminal is, for example, a wireless communication device that ensures portability and mobility, and includes all types of handheld-based wireless communication devices such as personal communication system (PCS), global system for mobile communication (GSM), personal digital cellular (PDC), personal handyphone system (PHS), personal digital assistant (PDA), international mobile telecommunication (IMT)-2000, code division multiple access (CDMA)-2000, W-code division multiple access (W-CDMA), a wireless broadband Internet (Wibro) terminal, and a smart phone, and wearable devices such as a watch, ring, bracelet, anklet, necklace, glasses, contact lenses, and a head-mounted-device (HMD).

In this specification, the line connection-type object prediction device may be implemented by including at least one of the aforementioned computer, server device, and portable terminal. For example, the line connection-type object prediction device may be one of a computer, a server device, and a portable terminal, and may also be implemented as a system in which the server device performs a prediction method in the form of a web service and provides the service result to the computer or/and the portable terminal.

The line connection-type object prediction device using the artificial intelligence according to an embodiment of the present disclosure may be characterized by receiving an image including a line connection-type object, detecting a feature map for a state in which objects are connected by lines in the image to perform artificial intelligence-based learning, training an artificial intelligence model that outputs object detection information for a state in which the objects are connected by the lines based on the detected feature map, and outputting line connection-type object prediction result information for an input image through the trained artificial intelligence model.

The line connection-type object prediction device using the artificial intelligence may reduce prediction errors and improve analysis accuracy and computation speed when the state of objects connected by lines is predicted.

Hereinafter, the line connection-type object prediction device using the artificial intelligence will be described in detail.

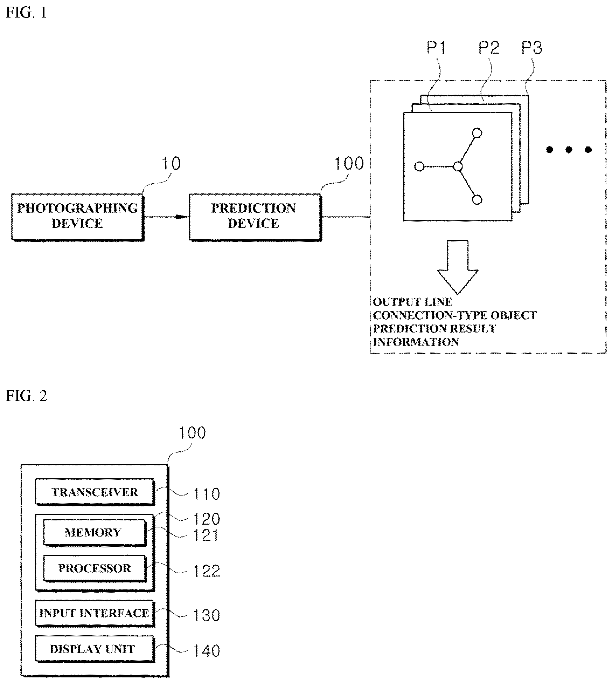

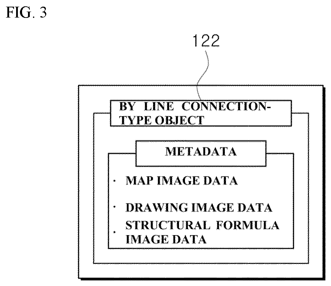

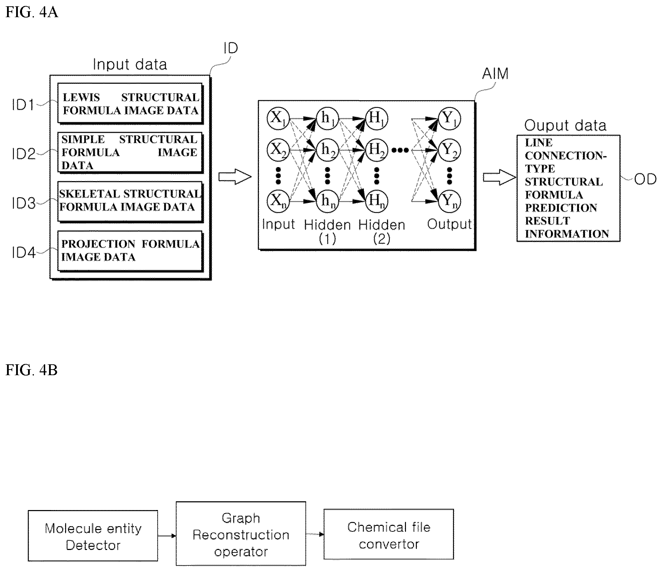

FIG. 1 is a diagram illustrating a line connection-type object prediction system using artificial intelligence according to an embodiment of the present disclosure. FIG. 2 illustrates the configuration of a line connection-type object prediction device of FIG. 1. FIGS. 3 and 4A to 4C are diagrams illustrating an example of a process for outputting line connection-type object prediction result information through the processor of FIG. 2.

Referring to FIGS. 1 to 4C, the line connection-type object prediction system using the artificial intelligence may include a photographing device 10 and a prediction device 100.

The photographing device 10 may acquire an image including the line connection-type object and transmit the same to the prediction device 100. The prediction device 100 may train an artificial intelligence model that performs object detection for images showing the bonding structure of compounds in various formats, thereby predicting (datafication) the line connection-type object. In this connection, the prediction device 100 may include a transceiver 110 and a controller 120.

Hereinafter, for explanation, the line connection-type object is limited to a compound. Accordingly, the prediction device 100 may recognize a molecular structure image of the compound and predict and datafy molecular structure information constructing the compound. In addition, the prediction device 100 may provide files in various compound naming formats or various structural formula images based on information on the datafied molecular structure.

The transceiver 110 may communicate with the photographing device 10. Herein, the transceiver 110 may receive images P1, P2, P3,... including the line connection-type object acquired from the photographing device 10. Herein, the line connection-type object may include at least one of a map, a drawing, or a structural formula. In this connection, the structural formula may include at least one of a Lewis structural formula, a simple structural formula, a skeletal structural formula, or a projection formula. For example, the skeletal structural formula may be a skeletal structural formula of a stereochemical type or a skeletal structural formula of an unspecified stereochemical type. For other examples, the projection formula may be a Newman projection formula, a Haworth projection formula, or a Fischer projection formula.

In other words, the prediction device 100 predicts the line connection-type object, which means recognizing an image representing the molecular structural formula of a compound and datafying the structural information of the molecule of the compound, and the prediction device 100 may convert the structural information of the molecule datafied as such in various formats described above or other formats and provide the same, or may utilize the same as a dataset for model training for various types of property analysis.

Herein, the structural information of the datafied molecule includes information on the position and positional relationship for nodes representing atoms and/or molecules, which are objects forming the molecular structural formula, and lines representing bonds, and thus may include all pieces of geometric information on a specific face of the compound represented by a molecular structural formula image.

In this connection, the transceiver 110 may include at least one of a wired communication module or a wireless communication module.

The wired communication module may include various wired communication modules such as a local area network (LAN) module, a wide area network (WAN) module, and a value-added network (VAN) module, as well as various cable communication modules such as a Universal Serial Bus (USB) module, a High-Definition Multimedia Interface (HDMI) module, a digital visual interface (DVI) module, a Recommended Standard 232 (RS-232), a power line communication module, and a plain old telephone service (POTS) module, and a processor or/and a memory for driving the same.

The wireless communication module may include, in addition to a Wi-Fi module and a WiBro module, wireless communication modules which support various wireless communication methods, such as a GSM module, a CDMA module, a W-CDMA module, a universal mobile telecommunications system (UMTS) module, a time-division multiple access (TDMA) module, a long-term evolution (LTE) module, 4G, 5G, and 6G, and a processor or/and a memory for driving the same.

The controller 120 may include a memory 121 and a processor 122.

The memory 121 may store data for an algorithm for controlling the operation of components within the device or a program reproducing the algorithm. The processor 122 may communicate with the memory 121 and perform the aforementioned operation using data stored in the memory 121. In this connection, the processor 122 may control operations related to line connection-type object prediction. Herein, the memory 121 and the processor 122 may be implemented as separate chips. In addition, the memory 121 and processor 122 may be implemented as a single chip.

The memory 121 may store data supporting various functions of the device and programs for the operation of components within the device, may store input/output data, and may store a number of application programs (or applications) driven on the device, data for the operation of the device, and instructions. At least some of these applications may be downloaded from external servers via wireless communication.

This memory 121 may include at least one type of storage medium among flash memory type, hard disk type, solid state disk (SSD) type, silicon disk drive (SDD) type, multimedia card micro type, card-type memory (for example, SD or XD memory), random access memory (RAM), static random access memory (SRAM), read-only memory (ROM), electrically erasable programmable read-only memory (EEPROM), programmable read-only memory (PROM), magnetic memory, magnetic disk, or optical disk.

The memory 121 may store the images P1, P2, P3,... including the line connection-type object. In addition, the memory 121 may store at least one of map image data, drawing image data, or structural formula image data, which are metadata for each line connection-type object. In this connection, the structural formula image data may include at least one of Lewis structural formula image data, simple structural formula image data, skeletal structural formula image data, or projection formula image data. The memory 121 may store the line connection-type object prediction result information output through an artificial intelligence model trained based on the object detection information.

The processor 122 may receive the images P1, P2, P3,... including the line connection-type object through the transceiver 110. In addition, the processor 122 may detect a feature map for a state in which objects are connected by lines in the images P1, P2, P3,... including the line connection-type object, in order to perform artificial intelligence-based training, and may generate an artificial intelligence model through training that may detect object detection information for a state in which nodes are connected by lines in the feature map. Accordingly, using the trained artificial intelligence model, the processor 122 may generate object detection information for a state in which the nodes are connected by the lines using a neural network-based object detector based on the feature map.

Herein, the object detection information includes information on atoms and atomic groups (hereinafter, "atoms"), and bonds therebetween, which are objects constructing the structure of a compound.

In addition, the object detection information is information that detects atoms and bonds for a molecular structure image, and thus may further include position information for each object. Here, the position information may mean information on the atoms connected by the detected bond, and may further include position information of each atom.

Furthermore, the object detection information may further include information on the type of each bond. For example, the object detection information may further include information on whether the bond is single, double, or triple, and classified into classes such as none or up, which may classify chiral and stereotypes.

For example, when the line connection-type object is a structural formula, the processor 122 may receive an image including the structural formula, detect a feature map from the image including the structural formula, and generate object detection information on a state in which atoms and bonds of atoms are connected by lines using an object detector in the feature map. In this connection, the processor 122 may first detect an atom that becomes a node and later detect a bond that becomes an edge.

Referring to FIG. 4A, the processor 122 may input image data in at least one format among Lewis structural formula image data ID1, simple structural formula image data ID2, skeletal structural formula image data ID3, and projection formula image data ID4 corresponding to structural formula image data among metadata, and train an artificial intelligence model (AIM) that has the object detection information as an output value, thereby outputting a result value of structural formula prediction result information OD of a recommended line connection-type. The AIM may be built and reinforcement trained as a learning data set using various pieces of Lewis structural formula image data ID1, various pieces of simple structural formula image data ID2, various pieces of skeletal structural formula image data ID3, and various pieces of projection formula image data ID4 using the object detector. In this connection, the memory 121 may store the line connection-type structural formula prediction result information OD trained and analyzed based on the AIM.

In order to predict the line connection-type object, an input interface 130 may input an image including the line connection-type object. Herein, a user may also directly input an image including the line connection-type object using the input interface 130. For example, the user may scan an image including the line connection-type object and directly input the image including the scanned line connection-type object using the input interface 130. The input interface 130 may be any input member capable of inputting an image including the line connection-type object.

The processor 122 may output the line connection-type object prediction result information that is trained and analyzed based on the AIM corresponding to the input information of the input interface 130. For example, the processor 122 may output the line connection-type structural formula prediction result information OD trained and analyzed based on the AIM.

A display unit 140 may also display the line connection-type object prediction result information output by the processor 122. For example, the display unit 140 may display the line connection-type structural formula prediction result information OD. In this connection, the display unit 140 may display line connection-type Lewis structural formula prediction result information, may display line connection-type simple structural formula prediction result information, may display line connection-type skeletal structural formula prediction result information, and may display line connection-type projection formula prediction result information.

In more detail, referring to FIG. 4B, the artificial intelligence model according to an embodiment may include an object detector (molecule entity detector) trained to detect objects (for example, nodes and edges) representing the structure of a compound of a molecular structural formula when a molecular structural formula image is input and output object detection information, an operator (graph reconstruction operator) that reconstructs graphical data based on the output object detection information, and a converter (chemical file convertor) that converts the reconstructed graphical data into various formats of compound names/structural formulas representing the molecular structure of the compound.

In an embodiment, the object detector, the operator, and the converter may each be implemented as separate modules. In other words, the artificial intelligence model may be implemented as a module constructing three different algorithms (for example, neural networks).

Recently, end-to-end vision tasks using natural language processing (NLP) artificial intelligence models are generally performed, but this is not suitable when accurate analysis of each precise object, such as molecular structure information of a compound, is required. For example, it is not a suitable method for generating the line connection-type object prediction result information by recognizing each and every detailed object representing atoms and bonds, such as a molecular structural formula image, and datafying molecular structural information.

In addition, when nodes, which are objects representing atoms in a molecular structural formula image, and lines, which are objects representing bonds, are clearly detected, the operator that reconstructs graphical data based thereon and the converter that converts the graphical data into various formats representing compounds correspond to technologies that have already been developed (for example, GNN, RDKit library).

Accordingly, when an object detector that accurately outputs object detection information forming a molecular structure from a molecular structural formula image is well implemented, datafication of molecular structure information of a compound may be sufficiently implemented using existing technology.

In addition, since there are so many pieces of chemical name/structural formula sample data in various formats matched with molecular structural formula images, it may be easy to build a training data set later.

Accordingly, in this embodiment, the object detector may be an artificial intelligence model optimized for detecting nodes representing atoms constituting a compound and lines representing bonds in a molecular structural formula image of the compound.

In an embodiment, the object detector may be an image detection model including a neural network trained with a training data set in which detection information of objects (nodes and edges) are mapped to molecular structural formula images. Herein, nodes are objects corresponding to atoms in the compound molecular structure, and edges are objects corresponding to bonds.

In addition, in an embodiment, the object detector may be an image detection/construction model that combines the aforementioned object detection model with the graph reconstruction operator, such that object detection information is output in the form of a graph.

In this connection, the graph representation of the compound mapped to the molecular structural formula image may be an image detection/construction model including a plurality of neural networks trained with a training data set.

For example, the object detector may be a model designed based on at least one object detection model among R-CNN, SPP-net, Fast R-CNN, Faster R-CNN, SSD, and YOLO. In other words, in an embodiment, the object detector may be implemented by including a neural network of the Fast R-CNN and Faster R-CNN series that has high object recognition accuracy and speed of detailed objects and accurately detects objects of node and edge classes.

In a compound, atoms are represented by element symbol characters and junctions and are detected as nodes, and the element symbol characters and junctions may be detected relatively accurately in a feature map. However, bonds are represented as lines (edges) between atoms, and when existing edge detectors are used, the detection accuracy is relatively low compared to nodes. Hence, there is an issue in that lines indicating double or triple bonds or lines incorrectly indicated as noise are not readily recognized.

The invention concept resides in that since bonds in compounds are formed between atoms, when objects are detected, nodes are detected first and then lines are detected using information on the detected nodes as additional information, lines may be detected accurately.

Accordingly, the object detector according to an embodiment of the present disclosure is an object detector having a structure that first detects a node and then additionally inputs information on the detected node to detect an edge.

In more detail, the object detector may detect an ROI for a node, detect edge information (LOI) representing a bond through information (feature map) on the ROI of the detected node and a molecular structural formula image, and then generate object detection information by bonding the ROI and LOI.

Herein, the object detection information may include node class information (atomic symbol, molecular formula of atomic group), node position information (for example, coordinate information of bounding box of each atom), edge class (for example, single, double, triple bond, mirror image, isomer), and edge position information (for example, edge coordinate information). This object detection information may be in a JSON format, with information for each list.

According to an embodiment, such object detection information includes information on the class and position of the node, and edge connected to each node, and thus includes all pieces of information on atoms and bonds needed for a molecular formula graph form, and thus may be information that serves as ae basis for conversion into a graphical representation of a molecular formula.

In other words, the object detection information may be converted into a graph of molecular structural formula Graph = N(nodes), E(edges) according to the graph reconstruction operator.

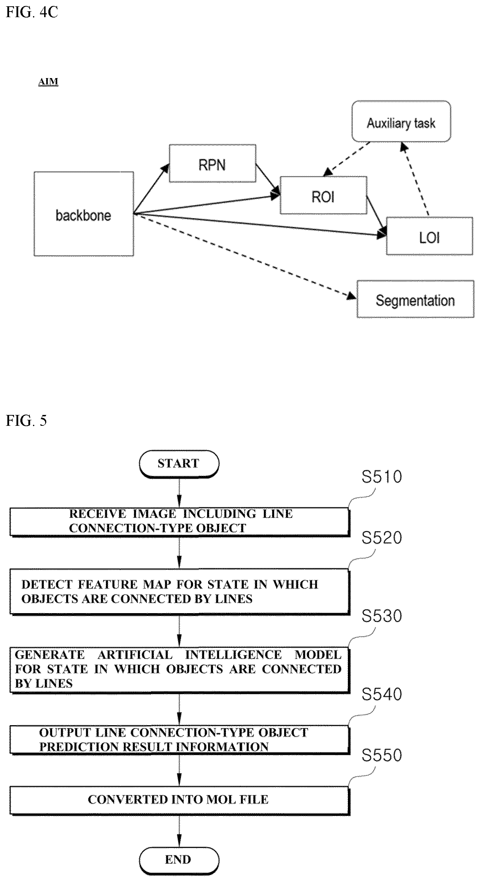

Referring to FIG. 4C, the object detector of one embodiment of the present disclosure may include a backbone that extracts an entire feature map from an image including a molecular structural formula image, a region proposal network (RPN) that extracts and proposes candidate regions (region proposals) in which an object may be included from the extracted entire feature map, an ROI module that determines an ROI for a node based on the entire feature map and the proposed plurality of candidate regions, and an LOI module (LOI) that determines an LOI for an edge based on the ROI of the node and the entire feature map.

More specifically, the backbone may extract the entire feature map for the entire image, including the molecular structural formula image.

In an embodiment, the backbone is a convolutional neural network (CNN) backbone, which may be implemented as ResNet or VGG.

Next, the RPN may extract and propose candidate regions for objects.

In detail, the RPN may extract candidate regions that may include nodes and propose a plurality of candidate feature maps for each candidate region.

To this end, the RPN may include an anchor generation layer that generates anchor boxes for the entire image, and a proposal layer that corrects the class scores of nodes and bounding boxes of candidate regions for each feature map of the generated anchor boxes.

In detail, the anchor generation layer may generate anchor boxes that are classified into different grid cells for the entire image size.

In addition, the proposal layer may extract a plurality of candidate regions by performing class score and bounding box correction (bounding box regressor) for each anchor box for the entire feature map.

Specifically, the proposed layer may extract the top N anchor boxes in terms of class scores after removing inappropriate objects by applying non-maximum suppression. Thereafter, regression coefficients are applied to the anchor boxes to help the anchor boxes better detect the location of the object, and finally candidate regions for the object may be detected.

Next, the ROI module may detect node detection information of nodes based on the proposed plurality of candidate regions. Herein, the node detection information may be a feature map for node regions corresponding to nodes (atomic symbols and junctions) in the molecular structural formula image. In addition, it may be node detection information generated by classifying the feature map for the node region and correcting the bounding box.

In detail, the ROI module may perform sample selection for nodes among candidate regions proposed through the proposal target layer. For example, the candidate regions matching the atomic symbol, which is a node that may correspond to an atom, the atomic group symbol (for example, a functional group), and the junction may be determined as samples. In other words, after the probability of a node among the plurality of candidate regions are calculated as a score, the candidate regions that exceed a preset reference may be determined as positive samples.

In addition, the ROI module may output a fixed-size feature map of a node for a positive sample of the node through a layer that performs RoI pooling based on the positive sample and the entire feature map determined as such.

In addition, the ROI (region of interest) module inputs the feature map of a node into a fully connected layer to obtain a feature vector, classifies the class using a softmax function or the like for the feature vector, and outputs each feature vector through bounding box correction (bounding box regressor) to ultimately output node detection information for the molecular structural formula image.

In addition, the LOI (lines of interest) module may output edge detection information representing a combination of molecular structural formulas based on the intermediate output data and/or the final output data and the entire feature map to the ROI module.

Herein, the edge detection information refers to an edge, which is a bond connecting different nodes, and therefore may include information defining the coordinates of the edge and the nodes placed at both ends of the edge.

These LOI modules may more accurately detect only the edges for the bonding when the intermediate data or/and final data of the ROI module and the entire feature map are input together, since the edges for the bond will be placed between different nodes.

These LOI modules may be implemented by modifying at least one edge detector structure, such as the Sobel, Prewitt, Scharr, and Canny Edge Detectors, using a CNN-based edge detector.

These LOI modules may also be CNN-based deep neural networks that include input layers, convolutional layers, pooling layers, and connected convolutional layers.

The object detector configured as such may output object detection information including an ROI for nodes and an LOI for edges when an entire image including a molecular structural formula image is input.

In a further embodiment, the object detector may further include a segmentation module that classifies the classes of the detected edges.

In other words, the object detector may further generate the segmentation module as an additional head module, and include the segmentation module that classifies edges as single (single bond), double (double bond), triple (triple bond), or mirror image (UP, down, none) for the entire feature map extracted from the backbone.

In addition, the object detector may further include edge class information to object detection information by adding a class for each edge in the LOI according to segmentation.

In this connection, the graphical reconstruction operator may reconstruct the graphical representation (Graph) = N(nodes), E(edges) = E(edges) Classification by adding the added edge class information.

In addition, the object detector may perform additional auxiliary tasks by feeding back the edge detection information of the LOI module to the ROI module, since node detection may be incorrect when the node detection information output from the ROI module does not match the edge detection information of the LOI module. In other words, the object detector may feed back data output from the LOI module, which is a lower layer, to the ROI module so that the ROI module may output node detection information again.

These object detectors may be trained based on a training data set in which a labeling task of nodes and a labeling task of edges in molecular structural formula images are performed.

In an embodiment, the object detector may train the ROI module, the RPN, and the backbone by applying a first loss function (detection loss) for node detection based on labels for the nodes in the molecular structural formula image.

In addition, the object detector may train the LOI module and backbone by applying a second loss function (WF loss) for edge detection based on the labels for edges in the molecular structural formula image.

In another embodiment, the object detector may be trained to output object detection information for the molecular structural formula of the compound more accurately by simultaneously applying node labels and edge labels to the molecular structural formula image based on a unifying loss for nodes and edges, thereby training the meaning of a compound structural formula between nodes representing atoms in the compound and edges representing bonds.

In addition, the processor 122 may generate object prediction result information including object detection information including node detection information and edge detection information.

In addition, the processor 122 may also generate object prediction result information by converting a molecular structural formula into graphical data based on graph object detection information.

Thereafter, the processor 122 may further convert the line connection-type object prediction result information into a Mol file (MDL Molfile). Herein, the processor 122 may convert line connection-type structural formula prediction result information into the Mol file. For example, the processor 122 may convert the line connection-type Lewis structural formula prediction result information into the Mol file, may convert line connection-type simple structural formula prediction result information into the Mol file, may convert line connection-type skeletal structural formula prediction result information into the Mol file, and may convert line connection-type projection formula prediction result information into the Mol file. In this connection, the MOL file may include molecular weight data information, atomic bond coordinates, and connection information in plain text format.

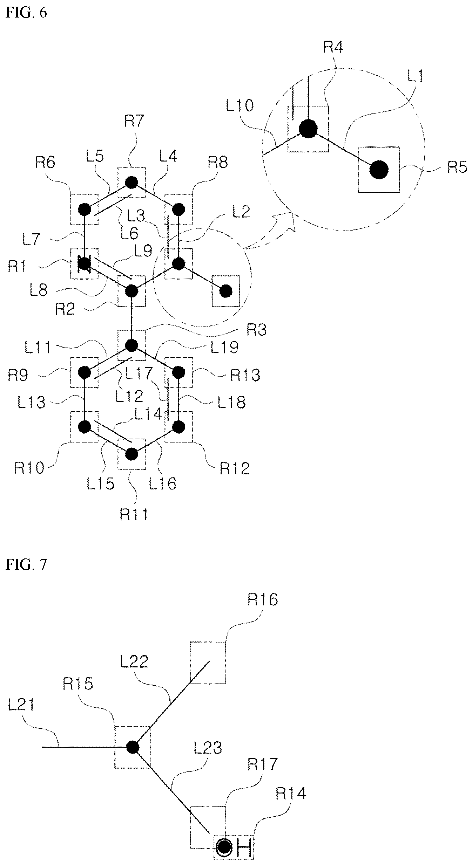

FIG. 5 is a flowchart illustrating a line connection-type object prediction method using artificial intelligence according to an embodiment of the present disclosure. FIG. 6 is a diagram illustrating an example of a process for detecting atoms, bonds, and lines in the processor of FIG. 2.

Referring to FIG. 5, the line connection-type object prediction method using artificial intelligence may include a reception stage (S510), a detection stage (S520), a generation stage (S530), an output stage (S540), and a conversion stage (S550).

The reception stage may receive images P1, P2, P3,... including the line connection-type object acquired from the photographing device 10 through the transceiver 110 (S510). Herein, the line connection-type object may include at least one of a map, a drawing, or a structural formula. In this connection, the structural formula may include at least one of a Lewis structural formula, a simple structural formula, a skeletal structural formula, or a projection formula. For example, the skeletal structural formula may be a skeletal structural formula of a stereochemical type or a skeletal structural formula of an unspecified stereochemical type. For other examples, the projection formula may be a Newman projection formula, a Haworth projection formula, or a Fischer projection formula.

The detection stage may detect a feature map for a state in which objects are connected by lines in images P1, P2, P3,... including the line connection-type object, in order to perform artificial intelligence-based learning through the processor 122 (S520). Herein, the processor 122 may detect a feature map for a state in which atoms and bonds of atoms are connected by lines in an image including a structural formula through the object detector.

For example, as illustrated in FIG. 6, the processor 122 may detect an atomic symbol R1 that becomes a node based on the ROI in an image including a skeletal structural formula, detect junctions R2 to R13 based on a Junction ROI, and detect edges L1 to L19 based on the LOI.

Herein, the processor 122 may simultaneously detect the atomic symbol R1 and the junctions R2 to R13 using the ROI module, and then detect the edge L1 to L19 using the LOI module.

In addition, the processor 122 may simultaneously detect the atomic symbol R1, the junctions R2 to R13, and the edges L1 to L19.

The generation stage may generate object detection information for a state in which objects are connected by lines based on a feature map through the processor 122 (S530). Herein, the processor 122 may generate object detection information for a state in which objects are connected by lines using the object detector based on the feature map. In this connection, the processor 122 may generate the object detection information for a state in which atoms and bonds of atoms are connected by lines using the object detector based on the feature map.

Herein, the processor 122 may train the AIM with a training data set including output values labeled with nodes (atoms) and edges (bonds) as input values of Lewis structural formula image data ID1, simple structural formula image data ID2, skeletal structural formula image data ID3, and projection formula image data ID4 corresponding to structural formula image data among metadata, and output a result value of recommended line connection-type structural formula prediction result information OD. The AIM may be built and reinforcement trained as a learning data set using various pieces of Lewis structural formula image data ID1, various pieces of simple structural formula image data ID2, various pieces of skeletal structural formula image data ID3, and various pieces of projection formula image data ID4 using the object detector. In this connection, the memory 121 may store the line connection-type structural formula prediction result information OD trained based on the AIM. For example, the line connection-type structural formula prediction result information OD may be the line connection-type Lewis structural formula prediction result information, the line connection-type simple structural formula prediction result information, the line connection-type skeletal structural formula prediction result information, and the line connection-type projection formula prediction result information.

FIGS. 7 to 13 are diagrams illustrating examples of a process for generating an artificial intelligence model based on linkage information between atoms, bonds, and lines in the processor of FIG. 2.

Referring to FIG. 7, in the case of a skeletal structural formula, the processor 122 may maintain labels for necessary junctions R15 based on linkage information between preset atomic symbols and junctions and edges, and generate object detection information after removing labels for unnecessary junctions R17, in order to increase the recognition rate of atomic symbols R14 while improving the computational speed. Herein, R15 and R16 may be either atomic symbols or junctions. In addition, L21 to L23 may be edges. In this connection, the linkage information may be atomic symbol type information, junction type information, edge connection information, or structural formula type information formed by atomic symbols, junctions, and edges. The processor 122 of an embodiment of the present disclosure is illustrated as a case of a skeletal structural formula for convenience of explanation in order to increase the recognition rate of the atomic symbol R14 while improving the computational speed, but the processor 122 of an embodiment of the present disclosure may also be applied in the case of a Lewis structural formula, a simple structural formula, or a projection formula.

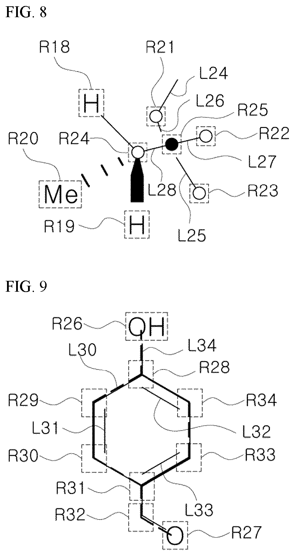

Referring to FIG. 8, in the case of a Lewis structure, the processor 122 may maintain levels for necessary junctions R21 to R24 based on linkage information between preset atomic symbols and junctions and edges, and generate object detection information after removing labels for unnecessary junctions R25, in order to increase the recognition rate of atomic symbols R18 to R20 and junctions R21 to R24 while improving the computational speed. In this connection, L24 to L28 may be edges. Herein, the linkage information may be atomic symbol type information, junction type information, edge connection information, or structural formula type information formed by atomic symbols, junctions, and edges. In this connection, the junction type information and the edge connection information may be defect information that is disconnected due to a junction. The processor 122 of an embodiment of the present disclosure is illustrated as a Lewis structural formula for convenience of explanation in order to increase the recognition rate of atomic symbols R18 to R20 and junctions R21 to R24 while improving the computational speed, but the processor 122 of an embodiment of the present disclosure may also be applied to a skeletal structural formula, a simple structural formula, or a projection formula.

Referring to FIG. 9, in the case of a skeletal structural formula, the processor 122 may predict the type of junctions R28 to R34 based on the linkage information between the preset atomic symbols R26, R27 and the edges, and then label the type of the predicted junction R28 to R34, and then generate object detection information, in order to increase the recognition rate of the junction R28 to R34 while improving the computational speed. In this connection, R26 and R27 may be atomic symbols, and L30 to L34 may be edges. Herein, the linkage information may be atomic symbol type information, junction type information, edge connection information, or structural formula type information formed by atomic symbols, junctions, and edges. In this connection, the junction type information and the edge connection information may be edge connection information forming a junction type by atomic symbol specie. The processor 122 of an embodiment of the present disclosure is illustrated as a skeletal structural formula for convenience of explanation in order to increase the recognition rate of junctions R28 to R34 while improving the computational speed, but the processor 122 of an embodiment of the present disclosure may also be applied to a Lewis structural formula, a simple structural formula, or a projection formula.

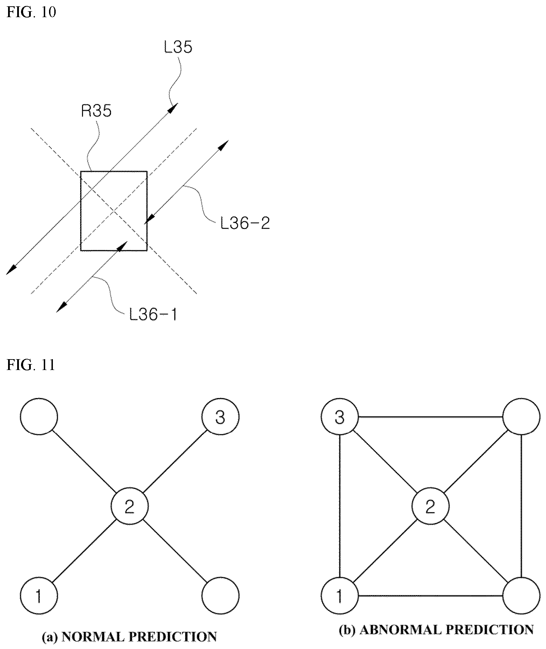

Referring to FIGS. 10 and 11, in the case of a skeletal structural formula, the processor 122 may predict the type of a junction R35 based on graph loss information, label the type of the predicted junction R35, and then generate object detection information using the object detector, in order to increase the recognition rate of the junction R35 while improving the computational speed. In this connection, L35 may be an edge in the case where the type for the junction R35 is normally predicted, and as illustrated in FIG. 11, the processor 122 may generate the object detection information corresponding to the case where the type for the junction R35 is normally predicted using the object detector. In addition, L36-1 and L36-2 may be edges in the case where the type for the junction R35 is abnormally predicted, and as illustrated in FIG. 11, the processor 122 may generate the object detection information corresponding to the case where the type for the junction R35 is abnormally predicted using the object detector.

To deal with this issue, an embodiment of the present disclosure may analyze the connection state between atomic symbols and junctions and edges based on graph loss information between atomic symbols and junctions and edges.

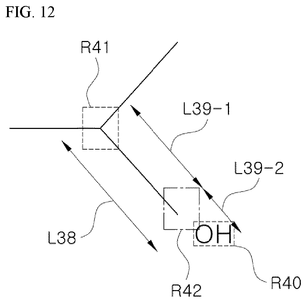

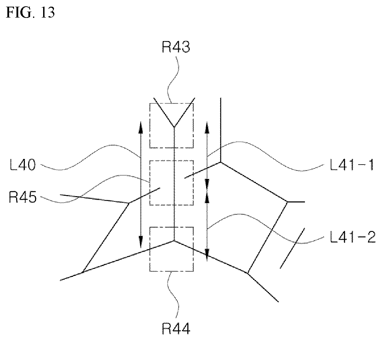

As illustrated in FIGS. 12 and 13, in the case of a skeletal structural formula, the processor 122 may analyze the connection state between the atomic symbol R40 and the junction R41 and R43, R44 and the edge L38, L39-1, L39-2 and L40, L41-1, L41-2 based on the graph loss information. Herein, the graph loss information may include at least one of the shape of an atom, the shape of a bond, the angle between lines, the length of a line, the shape of a line, and the shape of a graph.

For example, as illustrated in FIG. 12, the processor 122 may generate object detection information by maintaining labels for necessary junctions R40, R41 and removing labels for unnecessary junctions R42 based on at least one of the shape of an atom, the shape of a bond, the angle between lines, the length of a line, the shape of a line, and the shape of a graph between the atomic symbol R40 and the junction R41 and the edge L38 and L39-1, L39-2. In this connection, L38 may be the loss calculated only with true positive samples, and L39-1 and L39-2 may be the losses calculated incorrectly due to false positive samples.

For another example, as illustrated in FIG. 13, the processor 122 may maintain labels for necessary junctions R43, R44 and remove labels for unnecessary junctions R45 based on at least one of the shape of a bond between the junctions R43, R44 and the edges L40 and L41-1, L41-2, the angle between lines, the length of the lines, the shape of the lines, and the shape of the graph, and then generate object detection information. In this connection, L40 may be the loss calculated only with true positive samples, and L41-1 and L41-2 may be the losses calculated incorrectly due to false positive samples.

As illustrated in FIGS. 12 and 13, the processor 122 of an embodiment of the present disclosure is illustrated as a skeletal structural formula for convenience of explanation, but the processor 122 of an embodiment of the present disclosure may also be applied to a Lewis structural formula, a simple structural formula, or a projection formula.

The output stage may output line connection-type object prediction result information trained and analyzed based on object detection information through the processor 122 (S540). In this connection, in order to predict the line connection-type object, the input interface 130 may input an image including the line connection-type object. Herein, a user may also directly input an image including the line connection-type object using the input interface 130. For example, the user may scan an image including the line connection-type object and directly input the image including the scanned line connection-type object using the input interface 130.

The processor 122 may output the line connection-type object prediction result information that is trained and analyzed based on the AIM corresponding to the input information of the input interface 130. In this connection, even when input information including error information is input, the processor 122 may learn and analyze based on the AIM, and output corrected line connection-type object prediction result information based on the analysis result. For example, the processor 122 may output the line connection-type structural formula prediction result information OD trained and analyzed based on the AIM.

Herein, the display unit 140 may also display the line connection-type object prediction result information output by the processor 122. In this connection, the display unit 140 may display the line connection-type structural formula prediction result information OD. For example, the display unit 140 may display line connection-type Lewis structural formula prediction result information, line connection-type simple structural formula prediction result information, line connection-type skeletal structural formula prediction result information, and line connection-type projection formula prediction result information. The display unit 140 may display reliable line connection-type object prediction result information to a user based on the results accurately analyzed by the processor 122.

The conversion stage may further convert the line connection-type object prediction result information into a Mol file (MDL Molfile) through the processor 122 (S550). Herein, the processor 122 may convert line connection-type structural formula prediction result information into the Mol file. For example, the processor 122 may convert the line connection-type Lewis structural formula prediction result information into the Mol file, may convert line connection-type simple structural formula prediction result information into the Mol file, may convert line connection-type skeletal structural formula prediction result information into the Mol file, and may convert line connection-type projection formula prediction result information into the Mol file.

An embodiment of the present disclosure may improve the analysis speed of a structural formula while reducing the error of predicting a junction as an atomic symbol when a line connection-type object is a structural formula. In addition, an embodiment of the present disclosure may improve analysis accuracy and computational speed by using the junction algorithm of the junction and the detection algorithm of the atomic symbol together.

An embodiment of the present disclosure may be applied to a structural formula of an inorganic compound having the number of outermost electrons other than eight, and may output line connection-type structural formula prediction result information based on object detection information for the structural formula of the inorganic compound.

For convenience of explanation, an embodiment of the present disclosure has been illustrated as outputting line connection-type structural formula prediction result information based on object detection information, but an embodiment of the present disclosure is not limited thereto, and at least one of line connection-type map prediction result information and line connection-type drawing prediction result information required to distinguish between a line and an object may be additionally output.

At least one component may be added or deleted corresponding to the performance of the components illustrated in FIGS. 1 to 4C. In addition, it will be readily apparent to those skilled in the art that the relative positions of the components may be changed in response to the performance or structure of the system.

Although FIG. 5 illustrates that a plurality of stages are performed sequentially. However, this is merely illustrative of the technical idea of the present embodiment. Those skilled in the art to which an embodiment of the present disclosure pertains may apply various modifications and variations by changing and performing the order illustrated in FIG. 5 or performing one or more of stages among the plurality of stages in parallel without departing from the essential characteristics of an embodiment of the present disclosure. The embodiment in FIG. 5 is not limited to a time-series order.

The disclosed embodiments may be implemented in the form of a recording medium storing instructions executable by a computer. The instructions may be stored in the form of program code, which, when executed by a processor, may generate program modules to perform the operations of the disclosed embodiments. The recording medium may be implemented as a computer-readable recording medium.

Computer-readable recording media include all types of recording media that store instructions that may be deciphered by a computer. Examples thereof include a read only memory (ROM), a random access memory (RAM), a magnetic tape, a magnetic disk, a flash memory, and an optical data storage device.

As described above, the disclosed embodiments have been described with reference to the attached drawings. Those skilled in the art will appreciate that the present disclosure may be practiced in other forms than the disclosed embodiments without changing the technical spirit or essential features of the present disclosure. The disclosed embodiments are illustrative and should not be construed as limiting.

### [INDUSTRIAL APPLICABILITY]

The present disclosure relates to a prediction device and method for datafying a molecular structural formula of a compound in a molecular structural formula image using an artificial intelligence model including a deep network, and thus has industrial applicability.

## Claims

1. A line connection-type object prediction device using artificial intelligence, the device is **characterized by** comprising:
a memory; and
a processor electrically connected to the memory and controlling an operation related to line connection-type object prediction,
wherein the processor:
receives an image comprising a molecular structural formula image;
detects nodes corresponding to atoms of a molecular structural formula in the received image to generate node detection information;
detects edges corresponding to bonds of the molecular structural formula based on the node detection information and the received image to generate edge detection information; and
outputs prediction result information of the molecular structural formula image comprising the node detection information and the edge detection information.

2. The device of claim 1, wherein the processor outputs an entire feature map for the received image through a backbone network.

3. The device of claim 2, wherein the processor extracts a plurality of candidate regions (region proposals) predicted as the nodes based on the output entire feature map using a region proposal network (RPN).

4. The device of claim 3, wherein the processor classifies and detects the nodes based on the entire feature map and the extracted plurality of candidate regions using a region of interest (ROI) module and output the node detection information.

5. The device of claim 4, wherein the ROI module determines a positive sample for a node among the plurality of candidate regions through a proposal target layer.

6. The device of claim 5, wherein the ROI module performs RoI pooling based on the determined positive sample and the entire feature map to output a feature map of a fixed-size node for the positive sample.

7. The device of claim 6, wherein the ROI module inputs the feature map of the node into a fully connected layer to output a feature vector, classifies a class for the output feature vector, and performs bounding box regressor to output the node detection information for the molecular structural formula image.

8. The device of claim 7, wherein the processor detects an edge based on the node detection information and the entire feature map using a line of interest (LOI) module, which is a CNN-based edge detector, and output the edge detection information.

9. The device of claim 8, wherein the processor datafies a graphical representation of the molecular structural formula based on object detection information comprising the node detection information and the edge detection information through a graphical reconstruction operator, and output the prediction result information of the molecular structural formula image.

10. The device of claim 8, wherein the processor performs an auxiliary task of feeding back the edge detection information output from the LOI module to the ROI module to perform node detection again.

11. The device of claim 8, wherein the processor trains the LOI module, the ROI module, the region proposal network, and a backbone based on a training data set in which node labels and edge labels are set for training images comprising the molecular structural formula image.

12. The device of claim 11, wherein the processor trains the LOI module, the ROI module, the region proposal network, and the backbone by simultaneously applying the node labels and the edge labels to the molecular structural formula image based on a unifying loss for the nodes and the edges.

13. The device of claim 1, wherein the processor further outputs edge classification information by classifying the edges by class of the edges through a segmentation module, and further comprise the output edge classification information in the object detection information.

14. A molecular structural formula image prediction method using artificial intelligence performed by a device, the method comprising:
receiving an image comprising a molecular structural formula image;
detecting a feature map for a state in which objects are connected by lines in the image;
detecting nodes corresponding to atoms of a molecular structural formula in the received image to generate node detection information;
detecting edges corresponding to bonds of the molecular structural formula based on the node detection information and the received image to generate edge detection information; and
outputting prediction result information of the molecular structural formula image comprising the node detection information and the edge detection information.
